(19) Europäisches Patentamt
European Patent Office
Office européen des brevets

(11) Veröffentlichungsnummer: **0 149 760**
**B1**

(12) # EUROPÄISCHE PATENTSCHRIFT

(45) Veröffentlichungstag der Patentschrift: **23.08.89**

(51) Int. Cl.⁴: **C 07 H 15/04**, A 61 K 31/70

(21) Anmeldenummer: **84114306.8**

(22) Anmeldetag: **27.11.84**

(54) **Substituierte O-Acyl-Glycosylamide, Verfahren zu ihrer Herstellung sowie ihre Verwendung.**

(30) Priorität: **07.12.83 DE 3344257**

(43) Veröffentlichungstag der Anmeldung:
**31.07.85 Patentblatt 85/31**

(45) Bekanntmachung des Hinweises auf die Patenterteilung:
**23.08.89 Patentblatt 89/34**

(84) Benannte Vertragsstaaten:
**AT BE CH DE FR GB IT LI NL SE**

(56) Entgegenhaltungen:
**EP-A-0 091 645**

**CHEMICAL ABSTRACTS, Band 87, Nr. 25, 19. Dezember 1977, Seite 768, Zusammenfassung Nr. 201958p, Columbus, Ohio, US; E. MASCIORINI et al.: "Synthesis of some N-methyl-N-acyl-aldopyranosylamines and their per-O-acyl derivatives", & AN. QUIM. 1976, 72(11-12), 946-9**
**CHEMICAL ABSTRACTS, Band 89, Nr. 15, 9. Oktober 1978, Seite 624, Zusammenfassung Nr. 129847y, Columbus, Ohio, US; S. HIRANO et al.: "Preparation of some novel N-acyl derivatives of 2-acetamido-3,4,6-tri-O-acetyl-2-deoxy-beta-D-glucopyranosylamine", & CARBOHYDR. RES. 1978, 65(2), 307-10**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Krüger, Bernd- Wieland, Dr., Sillerstrasse 49, D-5600 Wuppertal 11 (DE)**
Erfinder: **Lockhoff, Oswald, Dr., Morgengraben 14, D-5000 Koeln 80 (DE)**
Erfinder: **Stadler, Peter, Dr., Am Ideck 8, D-5657 Haan 1 (DE)**
Erfinder: **Metzger, Karl Georg, Dr., Pahlkestrasse 75, D-5600 Wuppertal 1 (DE)**
Erfinder: **Optiz, Hans- Georg, Dr. Miles Laboratories, Inc., 2200 Powell Street P.O. Box 8817, Emeryville California 94662 (US)**
Erfinder: **Stünkel, Klaus Georg, Dr., Am Eckbush 55, D-5600 Wuppertal 1 (DE)**
Erfinder: **Zeiler, Klaus Georg, Dr., Elsbeeker Strasse 46, D-5620 Velbert 15 (DE)**

(56) Entgegenhaltungen: (Fortsetzung)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

**Beschreibung**

Die vorliegende Erfindung betrifft Verbindungen der allgemeinen Formel I

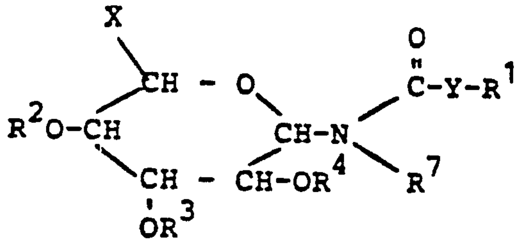

(I)

in welcher

X für Wasserstoff oder den Rest $-CH_2OR^5$ steht,
$R^2$, $R^3$, $R^4$, $R^5$ gleich oder verschieden sind und für Wasserstoff oder den Rest

$-\overset{\overset{O}{\|}}{C}-Z-R^6$ stehen,

Y, Z gleich oder verschieden sind und für Sauerstoff, Schwefel, N-H oder $CH_2$ stehen, und worin
$R^1$, $R^6$ und $R^7$ gleich oder verschieden sind und für einen gegebenenfalls substituierten Kohlenwasserstoffrest mit bis zu 50 Kohlenstoffatomen stehen,
mit der Maßgabe, daß mindestens einer der Reste $R^2$, $R^3$, $R^4$ und $R^5$ die Bedeutung

$-\overset{\overset{O}{\|}}{C}-Z-R^6$ hat

In der EP-91 645 werden analoge Verbindungen beschrieben, die aber nicht 0-acyliert sind.

Unter Kohlenwasserstoffrest in der Bedeutung der Reste $R^1$, $R^6$, $R^7$ wird erfindungsgemäß ein geradkettiger oder verzweigter Alkylrest, ein geradkettiger oder verzweigter, ein- oder mehrfach ungesättigter Alkenylrest, ein gesättigter oder ungesättigter alicyclischer Rest oder ein aromatischer Rest (Aryl) verstanden. Diese Bedeutungen können innerhalb desselben Restes $R^1$, $R^6$, $R^7$ auch gemeinsam auftreten, d.h. beispielsweise als Alkylcycloalkyl, Arylalkyl, Alkylaryl, Alkenylcycloalkyl, etc.

In den Resten $R^1$, $R^6$ und $R^7$ können auch einzelne, im allgemeinen bis zu 5, vorzugsweise 1, 2 oder 3 Methylen- oder Methingruppen durch O, S und/oder N ersetzt sein. Bei einer Unterbrechung der Kette durch N trägt dieser Stickstoff entweder H oder einen $C_1$-$C_{20}$-Alkylrest oder einen -CO-Alkylrest, wobei diese Alkylgruppe 1 - 20 C-Atome aufweist.

Bevorzugt stehen $R^1$, $R^6$, $R^7$ wahlweise für Alkyl- oder Alkenylreste mit 1 bis 21 Kohlenstoffatomen, vorzugsweise mit 9 bis 21 C-Atomen. Beispielhaft für gesättigte Reste seien hier genannt Methyl, Ethyl, Propyl, i-Propyl, Butyl, i-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Docosyl, Ethylpentyl, Methyldecyl, i-Propyldecyl, Methyltridecosyl, Eicosyl, Tetracosyl, Triacontyl, Pentahexadecyl, 1-Dodecylhexadecyl, 2-Dodecylhexadecyl, 3-Dodecylhexadecyl, 1-Hexadecyloctadecyl, 2-Hexadecyloctadecyl, 3-Hexadecyloctadecyl, 4-Hexadecyloctadecyl, 1-Octadecyleicosyl, 2-Octadecyleicosyl.

Ungesättigte Reste sind beispielsweise Ethenyl, Propenyl-1, Propenyl-2, i-Butenyl, Butenyl-1, Butenyl-2, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Decenyl, 5-Decenyl, 9-Decenyl, 8-Heptadecenyl, 1,3-Butadienyl, 1,3-Pentadienyl, 1,4-Pentadienyl, 2,4-Pentadienyl, 8,11-Heptadecandienyl, 8,11,14-Heptadecantrienyl. Im allgemeinen sind die längerkettigen, ungesättigten Reste bevorzugt, speziell die ein- oder zweifach ungesättigten Alkenyle mit 9 - 21 C-Atomen.

Die ungesättigten Kohlenwasserstoffreste können dabei als reine cis- oder trans-Isomere oder auch als Isomerengemische vorliegen.

Beispielhaft für Cycloalkyl seien genannt Cyclopentyl, Cyclohexyl, Decahydronaphthyl, Adamantyl.

Beispielhaft für Alkyl-cycloalkylreste seien genannt Methylcyclopentyl, Ethylcyclopentyl, n-Propylcyclopentyl, i-Propylcyclopentyl, Butylcyclopentyl, Octylcyclopentyl, Methylcyclohexyl, Ethylcyclohexyl, Propylcyclohexyl, Butylcyclohexyl, Hexylcyclohexyl, Decylcyclohexyl, Cyclopentylmethyl, Cyclopentylethyl, Cyclopentylpropyl, Cyclopentylbutyl, Cyclopentylpentyl, Cyclopentylhexyl, Cyclopentyloctyl, Cyclopentyldecyl, Cyclohexylmethyl, Cyclohexylethyl, Cyclohexylpropyl, Cyclohexylbutyl, Cyclohexylhexyl, Cyclohexyldecyl, Cyclopentyl, Cyclohexylethyl, Cyclohexylcyclopentylethyl und Cyclohexylcyclohexylethyl.

Beispielhaft für Aryl seien genannt Phenyl, Naphthyl, Diphenyl.

Beispiele für Aralkyl für $R^1$, $R^6$, $R^7$ sind Aryl-niedrigalkyl wie Benzyl, Phenethyl oder Phenylhexyl.

Die Reste $R^1$, $R^6$, $R^7$ können substituiert sein, im allgemeinen 1- bis 5-, vorzugsweise 1- bis 3-fach. Als Substituenten kommen vorzugsweise die folgenden in Betracht: Halogen, vorzugsweise F, Cl oder Br, Amino,

$C_1$-$C_6$-Alkylamino, Di-$C_1$-$C_6$-alkylamino, Oxo, OH, $C_1$-$C_6$-Alkoxy, SH, $C_1$-$C_6$-Alkylthio, $C_1$-$C_6$-Alkyl-COO und $C_1$-$C_6$-Alkyl-CO-NH.

Beispiele für Fälle, in denen die Kohlenwasserstoffreste $R^1$, $R^6$, $R^7$ durch O, S und N bzw. entsprechende Atomgruppierungen unterbrochen oder z. B. durch diese Atome enthaltende Gruppen oder durch Halogenatome substituiert sind, sind Methoxyethyl, Ethoxyethyl, n-Propoxyethyl, n-Butoxyethyl, i-Propoxyethyl, i-Butoxyethyl, sek.-Butoxyethyl, Methoxyethoxyethyl, Ethoxyethoxyethyl, Propoxyethoxyethyl, i-Propoxyethoxyethyl, n-Butoxyethoxyethyl, i-Butoxyethoxyethyl, sek.-Butoxyethoxyethyl, Methoxyethoxyethoxyethyl, Ethoxyethoxyethoxyethyl, n-Propoxyethoxyethoxyethyl, i-Propoxyethoxyethoxyethyl, n-Butoxyethoxyethoxyethyl, i-Butoxyethoxyethoxyethyl, sek.-Butyloxyethoxyethoxyethyl, wenn Y und/oder Z für Sauerstoff, Schwefel, N-H oder $CH_2$ stehen; Methoxyethoxy, Ethoxyethoxy, n-Propoxyethoxy, i-Propoxyethoxy, n-Butoxyethoxy, i-Butoxyethoxy, sek.-Butoxyethoxy, Methoxyethoxyethoxy, Ethoxyethoxyethoxy, n-Propoxyethoxyethoxy, i-Propoxyethoxyethoxy, n-Butoxyethoxyethoxy, i-Butoxyethoxyethoxy, sek.-Butoxyethoxyethoxy, wenn Y und /oder Z für $CH_2$ stehen; Hydroxyheptadecenyl, Oxobutyl, Amino-decyl-, N-Methylaminodecyl-, Fluormethyl-, β-Hydroxytridecyl- oder Mercaptoethylrest.

Die Verbindungen der Formel I enthalten mehrere chirale C-Atome und liegen als optische reine Diastereomere oder als Diastereomerengemische vor.

Die erfindungsgemäßen Verbindungen der Formel I sind also Carbonsäureamide bzw. N-alkylierte bzw. N-aralkylierte Carbonsäureamide, Carbamidsäure-, Thiocarbamidsäure- oder Harnstoffderivate, die an dem durch den obengenannten Rest $R^7$ substituierten Stickstoffatom zusätzlich N-glycosidisch, d.h. über das anomere Kohlenstoffatom gebunden einen einfachen Monosaccharidrest tragen, wobei eine oder mehrere Hydroxygruppen im Saccharidrest mit einem Acyl-, Alk(aryl)oxycarbonyl-, Alkyl(Aryl)thiocarbonyl- oder einem Carbamoylrest versehen sind.

Besonders bevorzugt sind Verbindungen, in denen nur einer der Reste $R^2$, $R^3$ $R^4$ oder $R^5$ die Bedeutung

O
||
-C-Z-$R^6$ hat,

mit der Maßgabe, daß die anderen Reste $R^2$, $R^3$, $R^4$ und/oder $R^5$ für Wasserstoff stehen und $R^6$ und Z die oben genannte Bedeutung haben.

Ganz besonders bevorzugt sind Verbindungen, in denen $R^2$, $R^3$ und $R^4$ für Wasserstoff stehen, und $R^5$ für den Rest

O
||
-C-Z-$R^6$ steht,

wobei Z und $R^6$ die obengenannte Bedeutung haben.

Als Beispiele seien genannt:

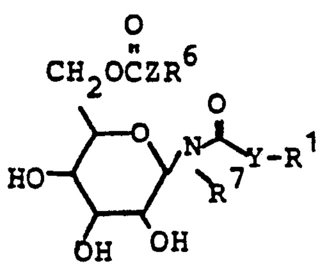

| Y | Z | $R^1$ | $R^6$ | $R^7$ |
|---|---|---|---|---|
| $CH_2$ | $CH_2$ | $(CH_2)_9CH_3$ | H | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | $OCH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | $CH_2$ | $(CH_2)_9CH_3$ | H | $(CH_2)_{11}CH_3$ |
| " | " | " | " | $(CH_2)_{13}CH_3$ |
| " | " | " | " | $(CH_2)_{15}CH_3$ |

| | | | | |
|---|---|---|---|---|
| " | " | " | " | $(CH_2)_{17}CH_3$ |
| " | " | " | $OCH_3$ | $(CH_2)_{11}CH_3$ |
| " | " | " | " | $(CH_2)_{13}CH_3$ |
| " | " | " | " | $(CH_2)_{15}CH_2$ |
| " | " | " | " | $(CH_2)_{17}CH_3$ |
| $CH_2$ | $CH_2$ | $(CH_2)_{11}CH_3$ | H | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | $OCH_3$ | $(CH_2)_{11}CH_3$ |
| " | " | " | " | $(CH_2)_{13}CH_3$ |
| | | | | $(CH_2)_{15}CH_3$ |
| " | " | " | " | $(CH_2)_{17}CH_3$ |
| O | " | " | H | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | $OCH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | $CH_2$ | $(CH_2)_{13}CH_3$ | H | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | $OCH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | " | " | H | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | $OCH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | $CH_2$ | $(CH_2)_{15}CH_3$ | H | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | $OCH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| O | " | " | H | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | " | $OCH_3$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | $CH_2$ | $(CH_2)_9CH_3$ | $OCH_2CH_2OC_4H_9$-n | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| | | | | $(CH_2)_{11}CH_3$ |
| " | " | " | " | $(CH_2)_{13}CH_3$ |
| " | " | " | " | $(CH_2)_{15}CH_3$ |

| | | | | |
|---|---|---|---|---|
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | $CH_2$ | $(CH_2)_9CH_3$ | $(OCH_2CH_2)_2OC_4H_9$-n | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | $CH_2$ | $(CH_2)_9CH_3$ | $(OCH_2CH_2)_2OC_4H_9$-n | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| $CH_2$ | O | $(CH_2)_9CH_3$ | $(CH_2CH_2O)_2$-$C_2H_5$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{11}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{13}CH_3$ | $(CH_2CH_2O)_2$-$C_2H_5$ | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |
| " | " | $(CH_2)_{15}CH_3$ | " | $(CH_2)_{11}CH_3$ |
| " | " | " | " | $(CH_2)_{13}CH_3$ |
| " | " | " | " | $(CH_2)_{15}CH_3$ |
| " | " | " | " | $(CH_2)_{17}CH_3$ |

Die Erfindung betrifft auch Verfahren zur Herstellung der Verbindungen der Formel I. Hierbei setzt man Verbindungen der Formel II

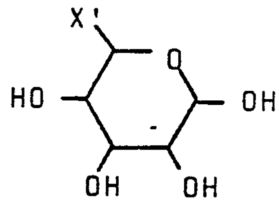 (II)

in der

X' für Wasserstoff oder $-CH_2OH$ steht

entweder in freier, d.h. ungeschützter Form, oder im Form geschützter, gegebenenfalls aktivierter Derivate zunächst mit einer Aminoverbindung $R^7-NH_2$, entweder in freier Form oder in Form eines geeigneten Säureadditionssalzes mit der vorstehend beschriebenen Bedeutung für $R^7$, um und acyliert anschließend das dabei erhaltene Glykosylamin mit einem - wie bei Acylierungsreaktionen üblich - aktivierten, an funktionellen Gruppen gegebenenfalls geschützten Carbonsäure-, Kohlensäure-, Thiokohlensäurederivat oder Isocyanat, spaltet in dem so erhaltenen Reaktionsprodukt gegebenenfalls vorhandene Schutzgruppen selektiv oder vollständig ab und setzt in einem zweiten Verfahrensschritt das so erhaltene, an mindestens einer Hydroxygruppe freie, d.h. ungeschützte, Zwischenprodukt mit einem - wie bei Acylierungsreaktionen üblich - aktivierten, an funktionellen Gruppen gegebenenfalls geschützten Carbonsäure-, Kohlensäure-, Thiokohlensäurederivat oder Isocyanat um. Nach Abspaltung gegebenenfalls vorhandener Schutzgruppen erhält man auf diese Weise die erfindungsgemäßen Verbindungen der Formel I, die, falls erforderlich, durch Chromatographie, Umkristallisieren, Extraktion o.ä. gereinigt werden können.

In einer bevorzugten Ausführungsform des erfindungsgemäßen Verfahrens wird in an sich bekannter Weise in einem ersten Verfahrensschritt der unblockierte Zucker der Formel II in einem geeigneten Lösungsmittel, oder auch ohne Lösungsmittel, gegebenenfalls in Gegenwart eines Katalysators bei Temperaturen zwischen 0°C und 80°C mit 1 bis 10 Äquivalenten des betreffenden Amins $R^7-NH_2$ umgesetzt und man erhält gewöhnlich in hohen Ausbeuten nach Aufarbeitung die betreffenden Glykosylamine als amorphe oder kristalline Feststoffe oder als zähe Sirupe.

Im zweiten Verfahrensschritt wird dann das Glykosylamin mit 1 bis 10 Äquivalenten eines Acylderivates der Formel

$R_1-Y-CO-X''$,

in der

$R_1$ und Y die obengenannte Bedeutung besitzen und

X'' Halogen oder eine bei Acylierungsreaktionen gebräuchliche Abgangsgruppe, vorzugsweise einen aktivierenden Esterrest, oder eine Gruppe $O-OC-Y-R_1$ mit der obigen Bedeutung für Y und $R_1$ bezeichnet,

bzw. 1 bis 10 Äquivalenten eines Isocyanates der Formel $R^1-NCO$ umsetzt, wobei man in einem organischen oder wäßrig-organischen Lösungsmittel bei Temperaturen zwischen -30°C und 80°C, gegebenenfalls in Gegenwart einer Base, arbeitet und nach beendeter Umsetzung das Reaktionsprodukt in üblicher Weise aufarbeitet.

In einem dritten Verfahrensschritt wird dann das so erhaltene, am Stickstoff umgesetzte Derivat, in geschützter oder ungeschützter Form mit 1 bis 10 Äquivalenten eines Acylderivates der Formel

$$R^6-Z-\overset{\overset{O}{\|}}{C}-X''' ,$$

in der

$R^6$ und Z die obengenannte Bedeutung besitzen und

X''' Halogen oder eine bei Acylierungsreaktionen gebräuchliche Abgangsgruppe, vorzugsweise einen aktivierenden Esterrest, oder eine Gruppe

$$O-\overset{\overset{O}{\|}}{C}-Z-R^6$$

mit der obigen Bedeutung für $R^6$ und Z bezeichnet

bzw. 1 bis 10 Äquivalenten eines Isocyanates der Formel $R^6-NCO$, umgesetzt, wobei man in einem organischen oder wäßrigorganischen Lösungsmittel bei Temperaturen zwischen -30°C und 80°C, gegebenenfalls in Gegenwart einer Base, arbeitet und nach beendeter Umsetzung das Reaktionsprodukt in üblicher Weise aufarbeitet.

Der erste Verfahrensschritt bei der Herstellung der erfindungsgemäßen Verbindungen der Formel I ist somit die Umsetzung eines Zuckers mit einem Amin des Typs $R^7$-$NH_2$ am anomeren Kohlenstoffatom unter Wasserabspaltung zu dem betreffenden Glykosylamin.

Amine $R^7$-$NH_2$, die bei Raumtemperatur flüssig sind, können mit dem Zucker direkt, d.h. ohne Lösungsmittel umgesetzt werden. Hierbei arbeitet man bei Temperaturen zwischen 0° C und 100° C vorzugsweise bei 25° C bis 70° C. Als Katalysatoren sind Mineralsäuren wie z. B. Salzsäure, Schwefelsäure oder Salpetersäure oder kurzkettige Carbonsäuren wie Essigsäure oder Propionsäure geeignet, die man in Mengen von 0,001 bis 0,05 Äquivalenten einsetzt.

In jedem Fall ist es möglich, und bei Aminen $R^7$-$NH_2$, die bei Raumtemperatur fest sind, auch zu bevorzugen, die Herstellung der Glykosylamine in Gegenwart eines Lösungsmittels durchzuführen. Man arbeitet dann vorzugsweise in Gegenwart eines unter den Reaktionsbebedingungen inerten Verdünnungsmittels, welches vorzugsweise so beschaffen ist, daß sich zumindest entweder die Reaktionspartner oder das Reaktionsprodukt in ihm lösen.

In Frage kommen Alkohole wie Methanol, Ethanol, Propanol-1 und Propanol-2, Ether wie Tetrahydrofuran und Dioxan, sowie auch Dimethylformamid, wobei - außer bei der Verwendung der Alkohole - der Zusatz von Wasser zu bevorzugen ist. Darüber hinaus ist vorzugsweise bei kurzkettigen Aminen $R^7$-$NH_2$ auch Wasser allein als Lösungsmittel geeignet. Es kann auch von Vorteil sein, die Alkanole im Gemisch mit Wasser zu verwenden.

Die Reaktionstemperaturen bei Verwendung von Lösungsmitteln bei der Herstellung der Glykosylamine liegen zwischen -10° C und 120° C vorzugsweise zwischen 30° C und 70° C.

Das betreffende Verdünnungsmittel kann wahlweise vor oder während der Reaktion zugegeben werden. Bei langkettigen Aminen $R^7$-$NH_2$ ist eine Zugabe vor der Reaktion zu bevorzugen.

Die wie vorstehend beschrieben hergestellten Glykosylamine kristallisieren entweder direkt oder nach Abkühlen aus und können durch Zusatz geeigneter, vorzugsweise weniger polarer Hilfslösungsmittel wie Aceton, Diethylether, Cyclohexan, Essigester oder Petrolether, gegebenenfalls unter Kühlung, ausgefällt oder zur Kristallisation gebracht werden, gegebenenfalls vorhandenes überschüssiges Amin $R^7$-$NH_2$ kann durch Waschen oder Umkristallisieren des Produktes auf an sich bekannte Weise entfernt werden.

Der zweite Verfahrensschritt bei der Herstellung der erfindungsgemäßen Verbindungen der Formel I ist die selektive N-Acylierung eines wie vorstehend beschrieben erhaltenen Glykosylamins mit einem Acylderivat der Formel $R_1$-Y-CO-X-"mit der vorstehend angegebenen Bedeutung von $R_1$, Y und X" oder einem Isocyanat der Formel $R^1$-NCO.

Der dritte Verfahrensschritt bei der Herstellung der erfindungsgemäßen Verbindungen der Formel I ist die selektive 0-Acylierung eines vorstehend beschriebenen geschützten oder ungeschützten N-acylierten Aminoglykosids mit einem Acylderivat der Formel

$$R^6\text{-Z-}\overset{\displaystyle O}{\overset{\|}{C}}\text{-X'''}$$

mit der vorstehend angegebenen Bedeutung von $R^6$, Z, X''' oder einem Isocyanat der Formel $R^6$-NCO.

Als an sich bekannte Carboxylderivate $R_1$-Y-CO-X" bzw.

$$R^6\text{-Z-}\overset{\displaystyle O}{\overset{\|}{C}}\text{-X'''}$$

sind zu bevorzugen Anhydride, aktivierte Ester und Säurehalogenide, vorzugsweise Chloride.

Diese Verbindungen werden vorzugsweise in Gegenwart eines Verdünnungsmittels mit den Glykosylaminen umgesetzt, in welchem die Reaktionspartner vollständig oder auch nur teilweise gelöst sind.

Es kommen organische oder anorganische Solventien in Frage, vorzugsweise solche, die unter den Reaktionsbedingungen Nebenreaktionen möglichst herabsetzen oder verhindern. Man kann sowohl in organischen Lösungsmitteln wie Ethern, z. B. Tetrahydrofuran und Dioxan, oder Alkoholen z. B. Ethanol und Propanol, oder Ketonen, z. B. Aceton oder Methylethylketon, oder in Dimethylformamid, Essigester oder Pyridin als auch in Mischungen dieser Lösungsmittel untereinander und/oder mit Wasser arbeiten. Die Verwendung von wasserfreien Solventien ist im allgemeinen zu bevorzugen.

Die Verbindungen $R^1$-Y-COX"/$R^6$ZCO-X''' bzw. $R^1$-NCO/$R^6$-NCO werden in 1 bis 10 Äquivalenten, bezogen auf Glykosylamin/Glykosylamid eingesetzt.

Die Reaktionen können vorzugsweise bei Verwendung von Säurehalogeniden und -anhydriden, in Gegenwart basischer Hilfsstoffe durchgeführt werden. Verwendet werden können alle in der organischen Synthese üblichen basischen Verbindungen wie z. B. tertiäre aliphatische oder auch aromatische Amine oder aber Alkali- und Erdalkalihydroxide bzw. -carbonate wie Natronlauge, Natriumcarbonat oder Calciumcarbonat.

Sie werden bei Temperaturen zwischen etwa -30° C und +80° C, vorzugsweise zwischen -10° C und +20° C durchgeführt.

In den Fällen, in denen eine selektive Umsetzung einer Hydroxylgruppe nicht durchgeführt werden kann, weil entweder eine weniger reaktive sekundäre Hydroxylgruppe des Zuckerrestes bei Vorhandensein einer reaktiveren primären Hydroxylgruppe mit dem Rest

O<br>
‖<br>
-C-Z-$R^6$

mit der obengenannten Bedeutung für Z und $R^6$ umgesetzt werden soll, oder aber eine sekundäre selektiv bei Vorhandensein anderer sekundärer Hydroxylgruppen mit dem Rest

O<br>
‖<br>
-C-Z-$R^6$

umgesetzt werden soll, ist der eigentlichen Acylierungsreaktion eine Reihe von Schutzgruppenoperationen vorzuschalten, bei der selektiv die mit dem Rest

O<br>
‖<br>
-C-Z$R^6$

umzusetzende Hydroxylgruppe am Ende der Blockierungsreaktionen frei, d.h. unsubstituiert, vorliegt. Die nicht umzusetzenden Hydroxylgruppen müssen also vor der Acylierung blockiert werden.

Geeignete Schutzgruppen für Zuckerderivate sind in der einschlägigen Literatur beschrieben (z. B. C.B. Reese, in Protecting Groups in org. Chem., 1973, P 95 - p 143; Plenum Press). Es können alle in der Zuckerchemie verwendeten Schutzgruppen und Kombinationen daraus benutzt werden.

Geeignete Schutzgruppen sind z. B. Ester wie Acetyl, Benzoyl, Pivaloyl, p-Methoxybenzoyl, Ether wie Benzyl, p-Methoxybenzyl, Allyl, 1-Propenyl, Alkylidenverbindungen wie Ethyliden, Isopropyliden, Benzyliden, Orthoester wie 1-Methoxy-ethyliden, 1-Ethoxy-ethyliden, Silylether wie Trimethylsilyl, t-Butyldimethylsilyl, Organometallverbindungen wie Borsäureester oder Zinnether oder Zinnketale wie Tributylstannyl oder Dibutylstannyliden.

Die durch diese Schutzgruppen blockierten Kohlenhydratderivate werden dann an der/den noch freien Hydroxylgruppe(n) in einem geeigneten Lösungsmittel mit der Gruppe

O<br>
‖<br>
$R^6$-Z-C-

umgesetzt. Geeignete Lösungsmittel und geeignete Verfahren zur Acylierung sind obengenannt.

Die auf diese Weise erhaltenen O-acylierten Amide, Harnstoffe, Carbamate bzw. Thiocarbamate werden nach an sich bekannten Verfahren in Form von kristallinen oder amorphen Feststoffen oder als zähe Sirupe isoliert und, wenn notwendig, durch Umkristallisation, Chromatographie, Extraktion usw. gereinigt.

Im Falle von Verbindungen mit geschützten Hydroxylgruppen im Glykosylteil können die Schutzgruppen in an sich bekannter Weise abgespalten werden.

Das folgende Formelschema soll eine der bevorzugten Ausführungsformen der erfindungsgemäßen Darstellung von Verbindungen der Formel I beispielhaft erläutern:

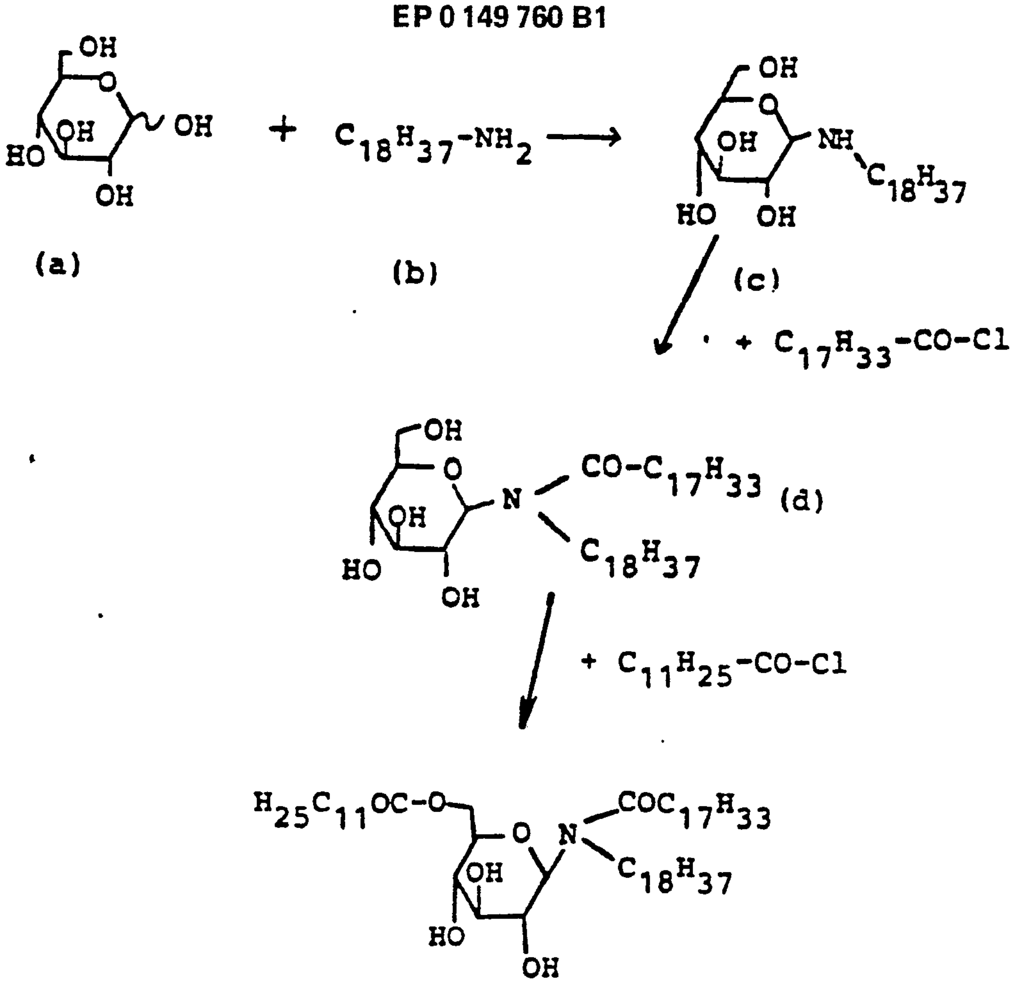


(a) (b) (c) (d)

(I)

Im ersten Verfahrensschritt wird Glucose (a) mit Octadecylamin (b) zu N-Octadecyl-β-D-glucopyranosylamin (c) umgesetzt, das im zweiten Verfahrensschritt mit Ölsäurechlorid zu N-Octadecyl-N-oleyl-β-D-glucopyranosylamin (d) acyliert wird. Im dritten Verfahrensschritt wird dann mit Dodecansäurechlorid in Position 6 O-acyliert und man erhält N-Octadecyl-N-oleyl-(6-lauroyl-β-D-glucopyranosyl)-amid (I).

Die Verbindungen der vorliegenden Erfindung weisen eine ausgeprägte Abwehr-steigernde Wirkung auf. Es wurde gefunden, daß die Verbindungsklasse die Antikörpersynthese des Immunsystems Antigen-spezifisch steigert und darüber hinaus die unspezifische wirtseigene Abwehr verstärkt. Diese Ergebnisse wurden anhand der nachfolgenden Versuchsanordnungen erhalten.

Steigerung der primären humoralen Immunität in vitro gegen Schaferythrozyten (SE).

Es ist experimentell möglich, die Entwicklung einer humoralen Immunantwort mit heterologen roten Blutzellen durch primäre Immunisation von Maus-Milzzellen in Suspensionskulturen in vitro einzuleiten (R.I. Mishell und R.W. Dutton, J. Exp. Med. 126, 423 (1967)). Hierzu wurden Balb/c Maus-Milzzellen für 5 Tage in Gegenwart von Antigen (SE) und der Testsubstanz kultiviert. Die Zellen werden geerntet, gewaschen und zusammen mit dem Antigen und Komplement in semi-solidem Agar ausplattiert und für 2 Stunden bei 37°C inkubiert (N.K. Jerne, A.A. Nordin und C. Henry, "Cell bound Antibodies", eds. Amos and Koprowski, Wistar Inst. Press, Philadelphia, USA, p. 109 (1963)). Die Antigen-Sensibilisierung von Maus-Lymphozyten in der Primärkultur resultiert in der Synthese und Freisetzung von Ak. Die spezifischen, sezernierten Antikörper binden sich an das SE-Antigen und lysieren diese Zellen durch die Gegenwart des Komplements (Plaque-Bildung). Substanzen der vorliegenden Verbindungsklasse (z. B. die der Beispiele 8, 9, 10, 12) sind in der Lage, dosisabhängig im Bereich 3 bis 100 µl/ml die Zahl der Antikörper-bildenden Zellen zu steigern.

Steigerung der primären humoralen Immunität in vivo gegen das lösliche Antigen Ovalbumin

NMRI-Mäuse wurden mit einer suboptimalen Antigendosis (1 µg/Tier, Tag 0) subcutan (s.c.) immunisiert. Bei suboptimaler Antigenstimulation wurde nur eine geringe Zahl von Lymphozyten der Tiere zur Antikörpersynthese angeregt. Die zusätzliche Behandlung der Tiere mit z. B. den erfindungsgemäßen Verbindungen der Beispiele 8 und 12 ist in der Lage, bei einer einmaligen Applikation von 3 bis 30 mg/kg subcutan den Antikörpertiter im Serum der Tiere signifikant zu steigern.

Die Antikörpertiterbestimmung erfolgte durch indirekte Hämagglutination am Tag 10. Der Effekt der Behandlung wird durch den geometrischen Mittelwert der $log_2$-Titer ausgedrückt.

Der immunstimulierende Effekt der genannten Verbindungen ist im Gegensatz zu anderen, z. B. bakteriellen Immunstimulantien wie LPS aus Gram-negativen Bakterien Antigen-abhängig, d.h. die Substanzen bewirken über raschenderweise nur in Verbindung mit einem antigenen Reiz (hier SE oder Ovalbumin) die Induktion der Antikörpersynthese. Sie haben im Gegensatz zu den erwähnten konventionellen Immunstimulantien keine mitogenen Eigenschaften.

**Verträglichkeit**

Obwohl Verbindungen der beschriebenen Art ihre potenzierende Wirkung an der Maus beispielsweise bereits nach einer Einzeldosis von 10 mg/kg i.p., oder peroral entfalten, werden auch bei Applikation von 100 mg/kg keine toxischen Effekte beobachtet. Die genannten Stoffe verfügen deshalb über eine gute Verträglichkeit.

Die erfindungsgemäßen Verbindungen haben die Fähigkeit, einerseits bei Mischung mit einem Antigen dessen Immunogenität zu erhöhen, andererseits bei systemischer Applikation die immunologische Reaktivität des behandelten Organismus zu steigern. Dabei sind die genannten Stoffe in der Lage, die für die Antikörperbildung verantwortlichen Lymphozyten zu aktivieren. Die neuen Verbindungen können somit als Adjuvantien in Mischung mit den Impfstoffen dazu benutzt werden, den Impferfolg und den durch Immunität vermittelten Infektionsschutz gegenüber bakteriellen, viralen oder parasitäten Erregern zu steigern.

Weiterhin eignen sich die beschriebenen Verbindungen in Mischung mit verschiedensten Antigenen als Adjuvantien bei der experimentellen und industriellen Herstellung von Antiseren für Therapie und Diagnostik. Darüber hinaus können die neuen Verbindungen auch ohne gleichzeitige Antigenzufuhr dazu benützt werden, bereits unterschwellig ablaufende Abwehrreaktionen bei Mensch und Tier zu fördern. Die Verbindungen eignen sich demnach besonders für die Stimulation der körpereigenen Abwehr, z. B. bei chronischen und akuten Infektionen oder bei selektiven (antigenspezifischen) immunologischen Defekten, sowie bei angeborenen, aber auch erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunsuppressiv wirkenden Stoffen auftreten. Die genannten Stoffe können somit vorzugsweise auch in Kombination mit antiinfektiösen Antibiotika, Chemotherapeutika oder anderen Heilverfahren verabreicht werden, um immunologischen Schädigungen entgegenzuwirken. Schließlich sind die beschriebenen Stoffe auch zur allgemeinen Prophylaxe von Infektionskrankheiten bei Mensch und Tier geeignet.

Die erfindungsgemäßen Verbindungen erhöhen die Überlebensrate im Tiermodell der akuten und chronischen bakteriellen Infektion.

**Versuchsbeschreibung**

Die erfindungsgemäßen Verbindungen können allein als Prophylaktikum zur Abwehr bestehender Infektionen oder in Kombination mit einer antibiotischen Therapie zur Steigerung der therapeutischen Wirkung von Antibiotika und Chemotherapeutika (z. B. Penicilline, Cephalosporine, Aminoglykoside etc.) bei infizierten Menschen und Tieren verwendet werden.

Es wurde gefunden, daß Infektionen der Maus mit pathogenen Keimen, die innerhalb von 24 bis 28 Stunden zum Tod der Versuchstiere führen, durch eine prophylaktische Behandlung - bevorzugt intraperitoneal - mit 1 bis. 80 mg/kg beispielsweise der erfindungsgemäßen Verbindungen der Beispiele 9, 10 und 12 therapiert werden können. Dies trifft für eine ganze Reihe grampositiver (z. B. Staphylokokken) und gramnegativer (z. B. E. coli, Klebsiella, Proteus, Pseudomonas) Krankheitserreger zu. Diese Aufzählung ist beispielhaft und keineswegs beschränkend aufzufassen. So überleben z. B. Mäuse die mit dem pathogenen Stamm Klebsiella 63 infiziert worden waren, nach Behandlung (z. B. 18 Stunden vor Infektion) mit 10 bis 40 mg/kg der erfindungsgemäßen Verbindung zu 40 bis 100 % diese Infektion während von den unbehandelten Kontrolltieren nur 0 bis 30 % überlebten.

In einem weiteren Versuchsmodell konnte gezeigt werden, daß die therapeutische Wirksamkeit von Antibiotika durch die erfindungsgemäßen Verbindungen gesteigert werden kann. So wurden Mäuse mit dem Stamm Pseudomonas W. infiziert. Diese Infektion führt bei den meisten Kontrolltieren innerhalb 24 Stunden zum Tode.

Eine weitere Gruppe wurde mit 4 mg/kg Sisomicin 30 Stunden post infectionem behandelt. Es konnte gezeigt werden, daß in der Versuchsgruppe die mit den erfindungsgemäßen Verbindungen 18 Stunden vor Infektion behandelt worden waren, die therapeutische Wirksamkeit des Sisomicins entscheidend verbessert werden konnte.

Die pharmazeutischen Präparate der vorliegenden Erfindung sind vorzugsweise Tabletten oder Gelatinekapseln, welche die Wirkstoffe zusammen mit Verdünnungsmitteln, z. B. Laktose, Dextrose, Saccharose, Mannit, Sorbit, Cellulose und/oder Schmiermitteln, z. B. Kieselerde, Talk, Stearinsäure oder Salze davon, wie Magnesium- oder Calciumstearat, und/oder Polyethylenglykol enthalten. Tabletten enthalten ebenfalls

Bindemittel, z. B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z. B. Stärken, Agar, Alginsäure oder ein Salz davon, wie Natriumalginat und/oder Brausemischungen, oder Adsorptionsmittel, Farbstoffe, Geschmackstoffe und Süßmittel. Injizierbare Präparate sind vorzugsweise isotonische wäßrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von etwa 0,1 % bis etwa 75 % insbesondere von etwa 1 % bis 50 % der genannten Aktivstoffe.

Die oral applizierten Präparate der vorliegenden Erfindung können auch mit einem gegen Magensaft beständigen Überzug versehen werden.

Die erfindungsgemäßen Verbindungen können als abwehrsteigernde und immunpotenzierende Mittel zur Behandlung von chronischen und akuten Infektionen (z. B. bakterielle, virale und parasitäre) und malignen Tumoren verwendet werden. Sie können ebenfalls als Adjuvantien bei der Vakzinierung, bei der Stimulierung von Phagocytose, bei der Dysregulierung des Abwehr- und Immunsystems verwendet werden.

## Beispiele

### Beispiel 1

N-Octadecyl-D-glucopyranosylamin

20 g Octadecylamin werden in 120 ml Ethanol gelöst und auf 70° erwärmt. Es werden 11 g wasserfreie D-Glucose zugesetzt. Nachdem sich eine klare Lösung gebildet hat wird noch 15 min. bei 70° weitergerührt. Es wurd auf 10° abgekühlt und 15 min. stehengelassen. Der gebildete Kristallbrei wird abgesaugt, zweimal mit Ethanol gewaschen und im Vakuum getrocknet.

Elementaranalyse:

| | | | |
|---|---|---|---|
| ber: | C 66,8 % | H 11,4 % | N 3,2 % |
| gef: | C 67,4 % | H 11,8 % | N 3,7 % |

### Beispiel 2

N-Glucopyranosyl-N-octadecyl-dodecansäureamid

10 g der Verbindung aus Beispiel 1 werden in 20 ml Tetrahydrofuran aufgeschlämmt und nach Zusatz von 10 g Soda tropfenweise mit 10 g Dodecansäurechlorid in 10 ml Tetrahydrofuran versetzt. Nach beendeter Reaktion (dünnschichtchromatographische Kontrolle auf Kieselgel 60 in Toluol-Isopropanol 6 : 1) wird der Feststoff abfiltriert, das Filtrat im Vakuum zum Sirup eingedampft und das Rohprodukt säulenchromatographisch auf Kieselgel 60 mit dem Elutionsmittel Toluol-Isopropanol 10 : 1 gereinigt.

$\alpha_D$ = 8° (c = 1,0 in Dioxan)

### Beispiel 3

N-Dodecyl-D-galactopyranosylamin

Herstellung gemäß Beispiel 1 aus D-Galactose und Dodecylamin.

Elementaranalyse

| | | | |
|---|---|---|---|
| ber: | C 62,2 % | H 10,7 % | N 4,0 % |
| gef: | C 62,5 % | H 10,2 % | N 4,4 % |

### Beispiel 4

N-Galactopyranosyl-N-dodecyl-octadecansäureamid

Herstellung gemäß Beispiel 2 aus 10 g der Verbindung gemäß Beispiel 3 und 16 g Stearinsäurechlorid.

$\alpha_D$ = 4,4° (c = 1,0 in Dichlormethan)

Rf-Wert = 0,23 in Toluol/n-Propanol 4 : 1

**Beispiel 5**

N-Octadecyl-N-(D-glucopyranosyl)-decylurethan

9 g der Verbindung aus Beispiel 1 werden in 160 ml Tetrahydrofuran und 40 ml Ethanol aufgeschlämmt und mit 9 g Natriumcarbonat versetzt. Zu dieser Suspension werden 5 g Chlorameisensäuredecylester, gelöst in 40 ml Tetrahydrofuran, während 20 min zugetropft. Nach beendeter Reaktion wird der Ansatz filtriert, der Filterrückstand mit Tetrahydrofuran nachgewaschen. Das Filtrat wird mit den Waschlösungen vereinigt und im Vakuum eingedampft. Der erhaltene Sirup wird chromatographisch gereinigt (Laufmittel Dichlormethan /Methanol, 20 : 1)

Rf-Wert 0,37 in $CH_2Cl_2/CH_3OH$ 10 : 1

Elementaranalyse

| | | | |
|---|---|---|---|
| ber: | C 68,3 % | H 11,3 % | N 2,3 % |
| gef: | C 68,4 % | H 11,6 % | N 2,4 % |

**Beispiel 6**

N-Octadecyl-N-(D-glucopyranosyl)-N'-dodecylharnstoff

9 g der Verbindung aus Beispiel 1 werden in 160 ml Tetrahydrofuran und 40 ml Ethanol aufgeschlämmt. Zu dieser Suspension werden 4,3 g Dodecylisocyanat, gelöst in 20 ml Tetrahydrofuran, während 20 min getropft. Nach beendeter Reaktion wird im Vakuum eingedampft und der erhaltene Sirup säulenchromatographisch gereinigt (Laufmittel Dichlormethan/Methanol, 15 : 1)

Rf-Wert: 0,33 in $CH_2Cl_2/CH_3OH$ 10 : 1

$\alpha_D$ = 7,4° (c = 1,04 in Dioxan)

**Beispiel 7**

N-Octadecyl-N-lauroyl-4-(3,6-Dioxodecoyl)-β-D-glucopyranosyl-amin

10 g (0,016 mol) der Verbindung aus Beispiel 2 werden in 100 ml Tetrahydrofuran gelöst, mit 3,5 g Benzaldehyddimethylacetal und 10 mg para-Toluolsulfonsäure versetzt und 1 h auf 70°C erwärmt. Nach dem Abkühlen auf Raumtemperatur wird mit Ionenaustauscher MP 500 (OH-Form) neutralisiert und im Hochvakuum eingeengt. Der erhaltene Sirup wird in 100 ml Tetrahydrofuran aufgenommen und nach Zugabe von 1,5 g Natriumhydrid und 3,5 ml Benzylbromid 1 h auf 40°C erwärmt. Nach dem Abkühlen auf Raumtemperatur werden 20 ml Methanol zugesetzt und 1 h nachgerührt. Der Ansatz wird vorsichtigt mit 10 ml Wasser versetzt und im Hochvakuum eingeengt. Der erhaltene Sirup wird in 100 ml Dichlormethan aufgenommen, zweimal mit je 20 ml Wasser extrahiert, über Magnesiumsulfat getrocknet, eingeengt im Vakuum und säulenchromatographisch gereinigt (Laufmittel Toluol/Essigester 20 : 1). Der erhaltene Sirup wird in 30 ml Tetrahydrofuran gelöst und nach Zugabe von 30 ml Eisessig/Wasser 10 : 1 1 h auf 70° erwärmt, auf Raumtemperatur abgekühlt und mehrfach mit Toluol im Hochvakuum eingedampft. Der erhaltene, chromatographisch einheitliche Sirup (7,8 g) wird in 100 ml Tetrahydrofuran gelöst, 30 min mit 400 mg Natriumhydrid bei 50° gerührt, auf 0° gekühlt und mit 1,8 g Benzylbromid versetzt. Nach 3 h wird auf Raumtemperatur erwärmt und über Nacht weitergerührt. Nach Zugabe von 5 ml Methanol wird 1 h nachgerührt, dann wird vorsichtig mit Wasser versetzt und eingedampft. Der erhaltene Rückstand wird in Dichlormethan und Wasser aufgenommen und die organische Phase mit Wasser extrahiert, getrocknet und eingeengt im Hochvakuum. Der Rückstand wird in 100 ml Tetrahydrofuran aufgenommen und die Lösung mit einer frisch hergestellten Lösung von 0,018 mol des gemischten Anhydrids aus Chlorameisensäureethylester und 3,6-Dioxodecansäure - gelöst in Tetrahydrofuran - versetzt.

Man rührt 12 h bei 20°C, filtriert die Reaktionsmischung und dampft das Filtrat im Vakuum zum Sirup ein, der dann säulenchromatographisch gereinigt wird (Petrolether: THF = 10 : 1).

Das erhaltene Hauptprodukt wird in 80 ml Tetrahydrofuran und 10 ml Eisessig gelöst und in Gegenwart von 2 g Palladiumkohle hydriert. Nach beendeter Wasserstoffaufnahme wird der Katalysator abfiltriert, das Filtrat im Vakuum zum Sirup eingedampft und mehrfach mit Toluol evaporiert.

Elementaranalyse

| | | | |
|---|---|---|---|
| ber: | C 68,44 % | H 11,10 % | N 1,81 % |
| gef: | C 68,3 % | H 10,9 % | N 1,8 % |

**Beispiel 8**

N-Octadecyl-N-3,6-dioxodecoyl-(6-stearyl-β-D-glucopyranosyl)-amin

1,2 g (0,004 mol) Octadecansäurechlorid und 2,0 g (0,0034 mol) N-Octadecyl-N-3,6-dioxodecoyl-β-D-glucopyranosylamin werden in 50 ml Tetrahydrofuran vorgelegt und bei 0°C mit 0,4 g Triethylamin (0,004 mol) versetzt. Man rührt 1 d bei 20°C, saugt den Feststoff ab und entfernt im Wasserstrahlvakuum das Lösungsmittel. Der Rückstand wird auf einer Säule mit 250 g Kieselgel im Laufmittelgemisch Toluol : Isopropanol = 10 : 1 chromatographiert und man erhält 1,9 g (65 % der Theorie) N-Octadecyl-N-3,6-dioxodecoyl-(6-stearyl-β-D-glucopyranosyl)-amin mit einem Rf-Wert von 0,296 (Toluol : Isopropanol = 6 : 1).

**Beispiel 9**

N-Octadecyl-N-lauroyl-6-[(2,2-dimethyl-propyl)-amino-carbonyl]-β-D-glucopyranosyl-amin

3,1 g (0,005 mol) N-Octadecyl-N-lauroyl-β-D-glucopyranosyl-amin und 20 ml DABCO in 40 ml Tetrahydrofuran werden bei 20°C innerhalb einer Stunde mit 0,7 g (0,0063 Mol) Neopentylisocyanat, gelöst in 20 ml Tetrahydrofuran versetzt. Man rührt 1 d bei 20°C und versetzt zur Vervollständigung der Reaktion erneut mit 0,2 g Isocyanat. Nach 2 d Rühren bei 20°C werden im Wasserstrahlvakuum flüchtige Bestandteile entfernt und der Rückstand an 200 g Kieselgel (Laufmittel Toluol : Isopropanol = 10 : 1) chromatographiert. Man erhält 1,4 g (39 % der Theorie) N-Octadecyl-N-lauroyl-6-[(2,2-dimethyl-propyl)amino-carbonyl]-β-D-glucopyranosyl-amin mit einem Rf-Wert von 0,34 (Toluol : Isopropanol = 6 : 1).

Analog erhält man

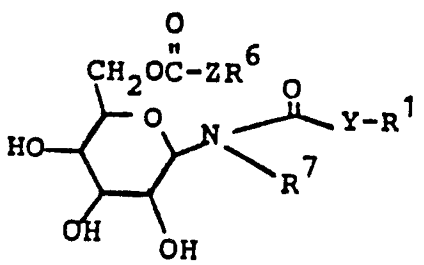

| Beispiel Nr. | Zucker | Y | Z | R1 | R6 | R7 | Rf (Tol: i-Prop = 6 : 1) |
|---|---|---|---|---|---|---|---|
| (10) | Glucose | $CH_2$ | $CH_2$ | $(CH_2)_9CH_3$ | H | $(CH_2)_{17}CH_3$ | 0,35 |
| (11) | ″ | $CH_2$ | $CH_2$ | $(CH_2)_9CH_3$ | $(CH_2)_{15}CH_3$ | ″ | 0,39 |
| (12) | ″ | $CH_2$ | $CH_2$ | $(CH_2)_9CH_3$ | $OCH_2CH_2OC_4H_9$-n | ″ | 0,35 |
| (13) | ″ | $CH_2$ | $CH_2$ | $(CH_2)_9CH_3$ | $(CH_2)_9CH_3$ | ″ | 0,36 |
| (14) | ″ | O | $CH_2$ | ″ | H | ″ | 0,35 |
| (15) | ″ | S | $CH_2$ | $(CH_2)_{17}CH_3$ | H | ″ | 0,34 |
| (16) | ″ | $CH_2$ | O | $(CH_2)_9CH_3$ | $CH_2CH_2OC_4H_9$-n | ″ | |
| (17) | ″ | $CH_2$ | S | ″ | ″ | ″ | |
| (18) | Galactose | $CH_2$ | $CH_2$ | ″ | $OCH_2CH_2OC_4H_9$-n | ″ | 0,36 |
| (19) | Mannose | $CH_2$ | $CH_2$ | ″ | ″ | ″ | 0,30 |
| (20) | Glucose | $CH_2$ | $CH_2$ | ″ | $OCH_3$ | ″ | 0,316 |
| (21) | ″ | $CH_2$ | $CH_2$ | ″ | $(OCH_2CH_2)_2OC_4H_9$-n | ″ | 0,323 |
| (22) | ″ | $CH_2$ | $CH_2$ | ″ | $(OCH_2CH_2)_4OCH_3$ | ″ | 0,173 |
| (23) | ″ | $CH_2$ | O | ″ | $CH_2CH_2OCH_2CH_2OC_2H_5$ | ″ | 0,302 |
| (24) | Mannose | $CH_2$ | $CH_2$ | $(CH_2)_{15}CH_3$ | $OCH_2CH_2OC_4H_9$-n | $(CH_2)_{13}CH_3$ | 0,32 |
| (25) | Galactose | $CH_2$ | $CH_2$ | $(CH_2)_{15}CH_3$ | $OCH_2CH_2OC_4H_9$-n | $(CH_2)_{11}CH_3$ | 0,34 |

**Patentansprüche** für die Vertragsstaaten BE, CH, DE, FR, GB, IT, LI, NL, SE

1. Verbindungen der allgemeinen Formel I

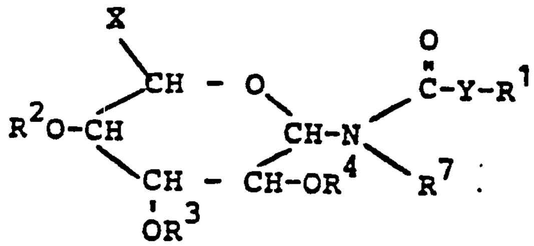

(I)

in der

X für Wasserstoff oder den Rest $-CH_2OR^5$ steht,
$R^2,R^3,R^4,R^5$ gleich oder verschieden sind und für Wasserstoff oder den Rest

$-\overset{\overset{O}{\|}}{C}-Z-R^6$ stehen

Y, Z gleich oder verschieden sind und für Sauerstoff, Schwefel, NH oder $CH_2$ stehen,
$R^1$, $R^6$, $R^7$ gleich oder verschieden sind und für einen gegebenenfalls substituierten Kohlenwasserstoffrest mit bis zu 50 C-Atomen stehen,
mit der Maßgabe, daß mindestens einer der Reste $R^2$, $R^3$, $R^4$ und $R^5$ die Bedeutung

$-\overset{\overset{O}{\|}}{C}-Z-R^6$ hat.

2. Verbindungen nach Anspruch 1, nach denen die Kohlenwasserstoffreste $R^1$, $R^6$ und $R^7$ bis zu 21 C-Atome aufweisen.

3. Verbindungen nach Anspruch 1, in denen die Kohlenwasserstoffreste $R^1$, $R^6$ und $R^7$ 9 - 21 C-Atome aufweisen.

4. Verbindungen nach den Ansprüchen 1 - 3, in denen $R^2$, $R^3$ und $R^4$ Wasserstoff darstellen und X die Bedeutung $CH_2OR^5$ mit $R^5 = COZR^6$ hat.

5. Verfahren zur Herstellung der Verbindungen gemäß Anspruch 1, dadurch gekennzeichnet, daß man eine Verbindung der Formel II

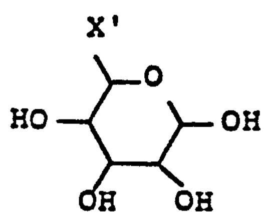

(II)

in der
X' für Wasserstoff oder $CH_2OH$ steht
entweder in freier, d.h. ungeschützter Form, oder in Form geschützter, gegebenenfalls aktivierter Derivate zunächst mit einer Aminoverbindung $R^7$-$NH_2$, entweder in freier Form oder in Form eines geeigneten Säureadditionssalzes mit der in Anspruch 1 genannten Bedeutung für $R^7$, umsetzt und anschließend das dabei erhaltene Glykosylamin mit einem bei Acylierungsreaktionen üblichen, aktivierten, an funktionellen Gruppen gegebenenfalls geschützten Carbonsäure-, Kohlensäure-, Thiokohlensäurederivat oder Isocyanat am Stickstoffatom acyliert, anschließend in dem so erhaltenen Reaktionsprodukt gegebenenfalls vorhandene Schutzgruppen selektiv oder vollständig abspaltet und in einem zweiten Verfahrensschritt das so erhaltene, an mindestens einer Hydroxygruppe freie, d.h. ungeschützte, Zwischenprodukt mit einem bei Acylierungsreaktionen üblichen, aktivierten, an funktionellen Gruppen gegebenenfalls geschützten Carbonsäure-, Kohlensäure-, Thiokohlensäurederivat oder Isocyanat umsetzt und gegebenenfalls vorhandene Schutzgruppen abspaltet.

6. Verfahren nach Anspruch 5, dadurch gekennzeichnet, daß man in einem ersten Verfahrensschritt einen unblockierten Zucker der Formel II in einem geeigneten Lösungsmittel, gegebenenfalls in Gegenwart eines Katalysators bei Temperaturen zwischen 0°C und 80°C mit 1 bis 10 Äquivalenten des Amins $R^7$-$NH_2$ zum Glykosylamin umsetzt, in einem zweiten Verfahrensschritt dann das Glykosylamin mit 1 bis 10 Äquivalenten

eines Acylderivates der Formel $R^1$-Y-CO-X", in der $R^1$ und Y die in Anspruch 1 genannte Bedeutung besitzen und X" Halogen oder eine bei Acylierungsreaktionen gebräuchliche Abgangsgruppe, vorzugsweise einen aktivierenden Esterrest, oder eine Gruppe O-OC-Y-$R^1$ mit der obigen Bedeutung für Y und $R^1$ bezeichnet, bzw. 1 bis 10 Äquivalenten eines Isocyanates der Formel $R^1$-NCO umsetzt, wobei man in einem organischen oder wäßrig-organischen Lösungsmittel bei Temperaturen zwischen -30° C und 80° C, gegebenenfalls in Gegenwart einer Base, arbeitet und in einem dritten Verfahrensschritt dann das so erhaltene, am Stickstoff umgesetzte Derivat, in geschützter oder ungeschützter Form mit 1 bis 10 Äquivalenten eines Acylderivates der Formel

O
||
$R^6$-Z-C-X''',

in der $R^6$ und Z die in Anspruch 1 genannte Bedeutung besitzen und X''' Halogen oder eine bei Acylierungsreaktionen gebräuchliche Abgangsgruppe, vorzugsweise einen aktivierenden Esterrest oder eine Gruppe

O
||
O-C-Z-$R^6$

mit der obigen Bedeutung für $R^6$ und Z bezeichnet, bzw. 1 bis 10 Äquivalenten eines Isocyanates der Formel $R^6$-NCO, umgesetzt werden, wobei man in einem organischen oder wäßrig-organischen Lösungsmittel bei Temperaturen zwischen -30° C und 80° C, gegebenenfalls in Gegenwart einer Base, arbeitet.

7. Arzneimittel enthaltend mindestens eine Verbindung gemäß Anspruch 1.

8. Verwendung der Verbindungen gemäß Anspruch 1 zur Herstellung von Arzneimitteln zur Bekämpfung von Krankheiten.

9. Verwendung der Verbindungen gemäß Anspruch 1 zur Herstellung von Arzneimitteln zur Steigerung der körpereigenen Abwehr.

10. Verwendung der Verbindungen gemäß Anspruch 1 zur Herstellung von Arzneimitteln zur Bekämpfung von Infektionskrankheiten.

**Patentansprüche** für den Vertagsstaat AT

1. Verfahren zur Herstellung von Verbindungen der allgemeinen Formel I

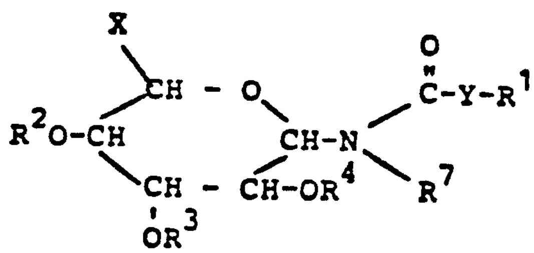

(I) ,

in welcher
X für Wasserstoff oder den Rest -$CH_2OR^5$ steht,
$R^2$,$R^3$,$R^4$,$R^5$ gleich oder verschieden sind und für Wasserstoff oder den Rest

O
||
-C-Z-$R^6$ stehen,

Y, Z gleich oder verschieden sind und für Sauerstoff, Schwefel, N-H oder $CH_2$ stehen, und worin

$R^1$, $R^6$ und $R^7$ gleich oder verschieden sind und für einen gegebenenfalls substituierten Kohlenwasserstoffrest mit bis zu 50 Kohlenstoffatomen stehen,

mit der Maßgabe, daß mindestens einer der Reste $R^2$, $R^3$, $R^4$ und $R^5$ die Bedeutung

O
||
$-C-Z-R^6$ hat,

dadurch gekennzeichnet, daß man eine Verbindung der Formel II

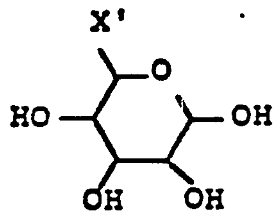

(II)

in der

X' für Wasserstoff oder $CH_2OH$ steht

entweder in freier, d.h. ungeschützter Form, oder in Form geschützter, gegebenenfalls aktivierter Derivate zunächst mit einer Aminoverbindung $R^7$-$NH_2$,

entweder in freier Form oder in Form eines geeigneten Säureadditionssalzes mit der oben genannten Bedeutung für $R^7$, umsetzt und anschließend das dabei erhaltene Glykosylamin mit einem bei Acylierungsreaktionen üblichen, aktivierten, an funktionellen Gruppen gegebenenfalls geschützten Carbonsäure-, Kohlensäure-, Thiokohlensäurederivat oder Isocyanat an Stickstoffatom acyliert, anschließend in dem so erhaltenen Reaktionsprodukt gegebenenfalls vorhandene Schutzgruppen selektiv oder vollständig abspaltet und in einem zweiten Verfahrensschritt das so erhaltene, an mindestens einer Hydroxygruppe freie. d.h. ungeschützte, Zwischenprodukt mit einem bei Acylierungsreaktionen üblichen, aktivierten, an funktionellen Gruppen gegebenenfalls geschützten Carbonsäure-, Kohlensäure-, Thiokohlensäurederivat oder Isocyanat umsetzt und gegebenenfalls vorhandene Schutzgruppen abspaltet.

2. Verfahren nach Anspruch 1, dadurch gekennzeichnet, daß man in einem ersten Verfahrensschritt einen unblockierten Zucker der Formel II in einem geeigneten Lösungsmittel, gegebenenfalls in Gegenwart eines Katalysators bei Temperaturen zwischen 0°C und 80°C mit 1 bis 10 Äquivalenten des Amins $R^7$-$NH_2$ zum Glykosylamin umsetzt, in einem zweiten Verfahrensschritt dann das Glykosylamin mit 1 bis 10 Äquivalenten eines Acylderivates der Formel $R^1$-Y-CO-X'', in der $R^1$ und Y die in Anspruch 1 genannte Bedeutung besitzen und X'' Halogen oder eine bei Acylierungsreaktionen gebräuchliche Abgangsgruppe, vorzugsweise einen aktivierenden Esterrest, oder eine Gruppe O-OC-Y-$R^1$ mit der obigen Bedeutung für Y und $R^1$ bezeichnet, bzw. 1 bis 10 Äquivalenten eines Isocyanates der Formel $R^1$-NCO umsetzt, wobei man in einem organischen oder wäßrig-organischen Lösungsmittel bei Temperaturen zwischen -30°C und 80°C, gegebenenfalls in Gegenwart einer Base, arbeitet und in einem dritten Verfahrensschritt dann das so erhaltene, am Stickstoff umgesetzte Derivat, in geschützter oder ungeschützter Form mit 1 bis 10 Äquivalenten eines Acylderivates der Formel

O
||
$R^6$-Z-C-X''',

in der $R^6$ und Z die in Anspruch 1 genannte Bedeutung besitzen und X''' Halogen oder eine bei Acylierungsreaktionen gebräuchliche Abgangsgruppe, vorzugsweise einen aktivierenden Esterrest oder eine Gruppe

O
||
O-C-Z-$R^6$

mit der obigen Bedeutung für $R^6$ und Z bezeichnet, bzw. 1 bis 10 Äquivalenten eines Isocyanates der Formel $R^6$-NCO, umsetzt, wobei man in einem organischen oder wäßrig-organischen Lösungsmittel bei Temperaturen zwischen -30°C und 80°C, gegebenenfalls in Gegenwart einer Base, arbeitet.

3. Verfahren nach Anspruch 1, dadurch gekennzeichnet, daß man die Reaktion mit einer bei Raumtemperatur flüssigen Aminoverbindung $R^7$-$NH_2$ unter Verzicht auf Verwendung eines Lösungsmittels bei Temperaturen zwischen 0 und 100°C durchführt.

4. Verfahren nach Anspruch 3, dadurch gekennzeichnet, daß man die Umsetzung mit der Aminoverbindung $R^7$-$NH_2$ bei Temperaturen von 25 - 70°C durchführt.

5. Verfahren nach Anspruch 3, dadurch gekennzeichnet, daß man die Umsetzung mit der Aminoverbindung $R^7$-$NH_2$ in Gegenwart von Mineralsäuren oder Carbonsäuren als Katalysatoren durchführt.

6. Verfahren nach Anspruch 5, dadurch gekennzeichnet, daß man die Katalysatoren in Mengen von 0,001 - 0,05 Äquivalenten, bezogen auf die Aminoverbindung, einsetzt.

7. Verfahren nach Anspruch 1, dadurch gekennzeichnet, daß man die Umsetzung mit einer Aminoverbindung

$R^7$-$NH_2$ in Gegenwart eines Lösungsmittels durchführt.

8. Verfahren nach Anspruch 7, dadurch gekennzeichnet, daß man die Reaktion in Methanol, Ethanol, Propanol-1, Propanol-2, Tetrahydrofuran, Dioxan oder Dimethylformamid durchführt.

9. Verfahren nach Anspruch 7, dadurch gekennzeichnet, daß man die Reaktion bei Temperaturen zwischen -10° C und 120° C durchführt.

10. Verfahren nach Anspruch 2, dadurch gekennzeichnet, daß man die Verbindungen $R^1$-Y-COX''/$R^6$ZCO-X''' bzw. $R^1$-NCO/$R^6$-NCO in 1 - 10 Äquivalenten, bezogen auf Glycosylamin/Glycosylamid einsetzt.

**Claims** for the contracting States BE, CH, DE, FR, GB, IT, LI NL, SE

1. Compounds of the general formula I

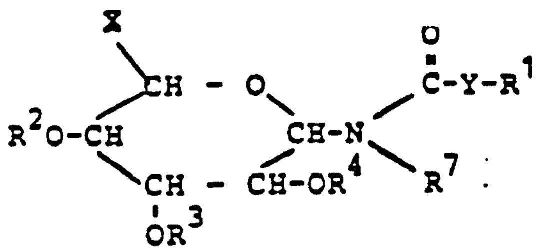

(I)

in which

X represents hydrogen or the radical -$CH_2OR^5$,

$R^2$, $R^3$, $R^4$ and $R^5$ are identical or different and represent hydrogen or the radical

O
||
-C-Z-$R^6$

Y and Z are identical or different and represent oxygen, sulphur, NH or $CH_2$, and

$R^1$, $R^6$ and $R^7$ are identical or different and represent an optionally substituted hydrocarbon radical having up to 50 C atoms,

subject to the proviso that at least one of the radicals $R^2$, $R^3$, $R^4$ and $R^5$ denotes

O
||
-C-Z-$R^6$.

2. Compounds according to Claim 1, in which the hydrocarbon radicals $R^1$, $R^6$ and $R^7$ contain up to 21 C atoms.

3. Compounds according to Claim 1, in which the hydrocarbon radicals $R^1$, $R^6$ and $R^7$ contain 9 - 21 C atoms.

4. Compounds according to Claims 1 - 3, in which $R^2$, $R^3$ and $R^4$ represent hydrogen and X denotes $CH_2OR^5$ in which $R^5$ is $COZR^6$.

5. Process for the preparation of the compounds according to Claim 1, characterised in that a compound of the formula

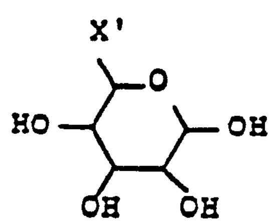

(II)

in which

X, represents hydrogen or $CH_2OH$

is reacted, either in the free, that is to say unprotected, form or in the form of protected, optionally activated derivatives, initially with an amino compound $R^7$-$NH_2$, either in the free form or in the form of a suitable acid addition salt, $R^7$ having the meaning mentioned in Claim 1, and the glycosylamine thus obtained is then acylated at the nitrogen atom by means of an activated carboxylic acid, carbonic acid or thiocarbonic acid derivative or isocyanate which is customary in acylation reactions and is, if appropriate, protected at functional groups, protective groups which may be present in the reaction product thus obtained are then split off

selectively or entirely, and, in a second process stage, the intermediate product thus obtained, which has at least one free, that is to say unprotected, hydroxyl group, is reacted with an activated carboxylic acid, carbonic acid or thiocarbonic acid derivative or isocyanate which is customary in acylation reactions and is, if appropriate, protected at functional groups, and protective groups which may be present are split off.

6. Process according to Claim 5, characterised in that, in a first process stage, an unblocked sugar of the formula II is reacted in a suitable solvent, if appropriate in the presence of a catalyst, at temperatures between 0°C and 80°C with 1 to 10 equivalents of the amine $R^7\text{-}NH_2$ to give the glycosylamine, the glycosylamine is then reacted in a second process stage with 1 to 10 equivalents of an acyl derivative of the formula $R^1\text{-}Y\text{-}CO\text{-}X''$ in which $R^1$ and Y have the meaning mentioned in Claim 1 and X'' denotes halogen or a leaving group which is customary in acylation reactions, preferably an activating ester radical,or a group $O\text{-}OC\text{-}Y\text{-}R^1$ in which Y and $R^1$ have the above meaning, or with 1 to 10 equivalents of an isocyanate of the formula $R^1\text{-}NCO$, the reaction being carried out in an organic or aqueous organic solvent at temperatures between -30°C and 80°C, if appropriate in the presence of a base, and, in a third process stage, the derivative thus obtained, which has been reacted at the nitrogen, is reacted in a protected or unprotected form with 1 to 10 equivalents of an acyl derivative of the formula

$$R^6\text{-}Z\text{-}\overset{\overset{\displaystyle O}{\|}}{C}\text{-}X'''$$

in which

$R^6$ and Z have the meaning mentioned in Claim 1 and

X''' denotes halogen or a leaving group which is customary in acylation reactions, preferably an activating ester radical or a group

$$O\text{-}\overset{\overset{\displaystyle O}{\|}}{C}\text{-}Z\text{-}R^6$$

in which

$R^6$ and Z have the above meaning,

or with 1 to 10 equivalents of an isocyanate of the formula $R^6\text{-}NCO$, the reaction being carried out in an organic or aqueous organic solvent at temperatures between -30°C and 80°C, if appropriate in the presence of a base.

7. Medicament containing at least one compound according to Claim 1.

8. The use of the compounds according to Claim 1 for the preparation of medicaments for combating diseases.

9. The use of the compounds according to Claim 1 for the preparation of medicaments for increasing the body's own defence mechanism.

10. The use of the compounds according to Claim 1 for the preparation of medicaments for combating infective diseases.

**Claims** for the contracting State AT

1. Process for the preparation of compounds of the general formula

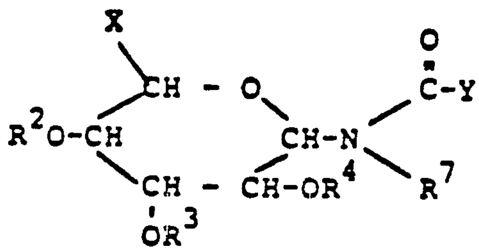

(I)

X represents hydrogen or the radical $-CH_2OR^5$,

$R^2$, $R^3$, $R^4$ and $R^5$ are identical or different and represent hydrogen or the radical

O
||
-C-Z-$R^6$,

Y and Z are identical or different and represent oxygen, sulphur, N-H or $CH_2$, and wherein

$R^1$, $R^6$ and $R^7$ are identical or different and represent an optionally substituted hydrocarbon radical having up to 50 carbon atoms,

subject to the proviso that at least one of the radicals $R^2$, $R^3$, $R^4$ and $R^5$

O
||
-C-Z-$R^6$,

characterised in that a compound of the formula II

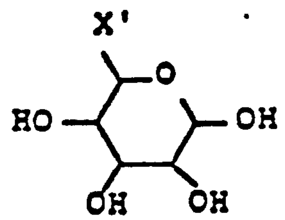

(II)

in which

X' represents hydrogen or $CH_2OH$,

is reacted, either in the free, that, is to say unprotected, form or in the form of protected, optionally activated derivatives, initially with an amino compound $R^7$-$NH_2$, either in the free form or in the form of a suitable acid addition salt, $R^7$ having the meaning mentioned above, and the glycosylamine thus obtained is then acylated at the nitrogen atom by means of an activated carboxylic acid, carbonic acid or thiocarbonic acid derivative or isocyanate which is customary in acylation reactions and is, if appropriate, protected at functional groups, protective groups which may be present in the reaction product thus obtained are then split off selectively or entirely, and, in a second process stage, the intermediate product thus obtained, which has at least one free, that is to say unprotected, hydroxyl group, is reacted with an activated carboxylic acid, carbonic acid or thiocarbonic acid derivative or isocyanate which is customary in acylation reactions and is, if appropriate, protected at functional groups, and protective groups which may be present are split off.

2. Process according to Claim 1, characterised in that, in a first process stage, an unblocked sugar of the formula II is reacted in a suitable solvent, if appropriate in the presence of a catalyst, at temperatures between 0°C and 80°C with 1 to 10 equivalents of the amine R'-$NH_2$ to give the glycosylamine, the glycosylamine is then reacted in a second process stage with 1 to 10 equivalents of an acyl derivative of the formula $R^7$-Y-CO-X" in which $R^1$ and Y have the meaning mentioned in Claim 1 and X" denotes halogen or a leaving group which is customary in acylation reactions, preferably an activating ester radical, or a group O-OC-Y-$R^1$ in which Y and $R^1$ have the above meaning, or with 1 to 10 equivalents of an isocyanate of the formula $R^1$-NCO, the reaction being carried out in an organic or aqueous organic solvent at temperatures between -30°C and 80°C, if appropriate in the presence of a base, and, in a third process stage, the derivative thus obtained, which has been reacted at the nitrogen, is reacted in a protected or unprotected form with 1 to 10 equivalents of an acyl derivative of the formula

O
||
$R^6$-Z-C-X'''

in which

$R^6$ and Z have the meaning mentioned in Claim 1 and

X''' denotes halogen or a leaving group which is customary in acylation reactions, preferably an activating ester radical or a group

O
||
O-C-Z-$R^6$

in which

$R^6$ and Z have the above meaning,

or with 1 to 10 equivalents of an isocyanate of the formula $R^6$-NCO,

the reaction being carried out in an organic or aqueous organic solvent at temperatures between -30°C and 80°C, if appropriate in the presence of a base.

3. Process according to Claim 1, characterised in that the reaction is carried out at temperatures between 0 and 100°C with an amino compound $R^7$-$NH_2$ which is liquid at room temperature, the use of a solvent being dispensed with.

4. Process according to Claim 3, characterised in that the reaction with the amino compound $R^7$-$NH_2$ is carried out at temperatures of 25 - 70°C.

5. Process according to Claim 3, characterised in that the reaction with the amino compound $R^7$-$NH_2$ is carried out in the presence of mineral acids or carboxylic acids as catalysts.

6. Process according to Claim 5, characterised in that the catalysts are employed in amounts of 0.001 - 0.05 equivalent, relative to the amino compound.

7. Process according to Claim 1, characterised in that the reaction with an amino compound $R^7$-$NH_2$ is carried out in the presence of a solvent.

8. Process according to Claim 7, characterised in that the reaction is carried out in methanol, ethanol, 1-propanol, 2-propanol, tetrahydrofuran, dioxane or dimethylformamide.

9. Process according to Claim 7, characterised in that the reaction is carried out at temperatures between -10°C and 120°C.

10. Process according to Claim 2, characterised in that the compounds $R^1$-Y-COX''/$R^6$ZCO-X''' or $R^1$-NCO/$R^6$-NCO are employed in 1 - 10 equivalents, relative to glycosylamine/glycosylamide.

**Revendications** pour Etats Contractants: BE, CH, DE, FR, GB, IT, LI, NL, SE.

1. Composés de formule générale I

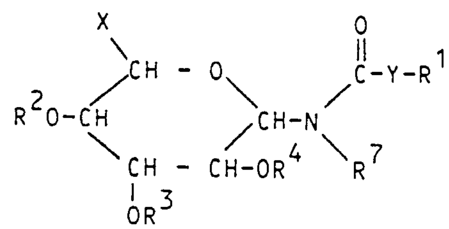

(I)

dans laquelle

X représente un atome d'hydrogène ou le reste -$CH_2OR^5$, les symboles

$R^2$, $R^3$, $R^4$ et $R^5$ représentent des restes identiques ou différents et représentent chacun un atome d'hydrogène ou le reste

O
||
-C-Z-$R^6$,

Y et Z sont identiques ou différents et représentent chacun un atome d'hydrogène ou de soufre ou un groupe -NH ou $CH_2$, les symboles

$R^1$, $R^6$ et $R^7$ sont identiques ou différents et représentent chacun un reste d'hydrocarbure, éventuellement substitué, comportant jusqu'à 50 atomes de carbone,

à la condition qu'au moins l'un des restes $R^2$, $R^3$, $R^4$ et $R^5$ représente(nt)

O
||
-C-Z-$R^6$.

2. Composés selon la revendication 1, dans lesquels les restes d'hydrocarbure $R^1$, $R^6$ et $R^7$ présentent jusqu'à 21 atomes de carbone.

3. Composés selon la revendication 1, dans lesquels les restes d'hydrocarbure $R^1$, $R^6$ et $R^7$ présentent 9 à 21 atomes de carbone.

4. Composés selon les revendications 1 à 3, dans lesquels $R^2$, $R^3$, $R^4$ représentent chacun un atome d'hydrogène, et X représente $CH_2OR^5$, dans lequel $R^5$ est un reste $COZR^6$.

5. Procédé pour préparer les composés selon la revendication 1, caractérisé en ce qu'on fait réagir un composé de formule II

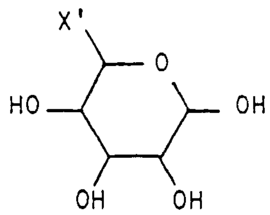

(II)

dans laquelle

X' représente un atome d'hydrogène ou le groupe $CH_2OH$,

sous forme libre c'est-à-dire non protégée, ou sous forme de dérivés protégés, éventuellement activés, tout d'abord avec un amine $R^7\text{-}NH_2$, sous forme libre ou sous forme d'un sel convenable d'addition d'acide, ayant pour $R^7$ le sens indiqué à la revendication 1, puis l'on soumet la glycosylamine ainsi obtenue à acylation sur l'atome d'azote à l'aide d'un dérivé d'acide carboxylique, d'acide carbonique ou d'acide thiocarbonique ou d'un isocyanate, usuels dans les réactions d'acylation, activés, et dont les groupes fonctionnels sont éventuellement protégés, puis l'on enlève, dans le produit réactionnel ainsi obtenu, sélectivement ou entièrement les groupes protecteurs éventuellement présents et, dans une seconde étape opératoire, on fait réagir le produit intermédiaire ainsi obtenu, comportant au moins un groupe hydroxyle libre, c'est-à-dire non protégé, avec un dérivé d'acide carboxylique, d'acide carbonique ou d'acide thiocarbonique ou avec un isocyanate, usuels dans les réactions d'acylation, activés et dont les groupes fonctionnels sont éventuellement protégés, et l'on sépare ou enlève les groupes protecteurs éventuellement présents.

6. Procédé selon la revendication 5, caractérisé en ce que, dans une première étape opératoire, on fait réagir un sucre non bloqué, de formule II, en opérant dans un solvant convenable, éventuellement en présence d'un catalyseur à des températures comprises entre 0°C et 80°C, avec 1 à 10 équivalents de l'amine $R^7\text{-}NH_2$ pour obtenir une glycosylamine; dans une seconde étape opératoire, on fait réagir ensuite la glycosylamine avec 1 à 10 équivalents d'un dérivé acylé de formule $R^1\text{-}Y\text{-}CO\text{-}X''$ (dans laquelle $R^1$ et Y ont le sens indiqué à la revendication 1, et X'' représente un atome d'halogène ou un groupe partant usuel dans les réactions d'acylation, avantageusement un reste ester à rôle d'activation, ou un groupe $O\text{-}OC\text{-}Y\text{-}R^1$ ayant le sens ci-dessus pour Y et $R^1$), ou bien avec 1 à 10 équivalents d'un isocyanate de formule $R^1\text{-}NCO$, en travaillant dans un solvant organique ou hydro-organique, à des températures comprises entre -30°C et 80°C, éventuellement en présence d'une base, et, dans une troisième étape opératoire, on fait ensuite réagir le dérivé ainsi obtenu, ayant réagi au niveau de son azote, sous forme protégée ou non protégée, avec 1 à 10 équivalents d'un dérivé acylé de formule

$$R^6\text{-}Z\text{-}\overset{\overset{\displaystyle O}{\|}}{C}\text{-}X''',$$

dans laquelle

$R^6$ et Z ont le sens indiqué à la revendication 1, et

X''' représente un atome d'halogène ou un groupe partant, usuel dans les réactions d'acylation, avantageusement un reste ester à rôle d'activation ou un groupe

$$O\text{-}\overset{\overset{\displaystyle O}{\|}}{C}\text{-}Z\text{-}R^6$$

dans lequel

les symboles $R^6$ et Z ont le sens ci-dessus, ou avec 1 à 10 équivalents d'un isocyanate de formule $R^6\text{-}NCO$, et l'on travaille alors dans un solvant organique ou hydro-organique, à des températures comprises entre -30°C et -80°C, éventuellement en présence d'une base.

7. Médicament contenant au moins un composé selon la revendication 1.

8. Utilisation des composés selon la revendication 1 pour préparer des médicaments destinés à combattre des maladies.

9. Utilisation des composés selon la revendication 1 pour préparer des médicaments destinés à augmenter la défense endogène.

10. Utilisation des composés selon la revendication 1 pour préparer des médicaments destinés à lutter contre des maladies infectieuses.

**Revendications** pour Etat Contractant: AT

1. Procédé pour préparer, des composés de formule générale I

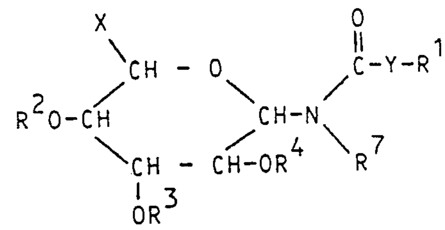

(I)

dans laquelle

X représente un atome d'hydrogène ou le reste $-CH_2OR^5$,

$R^2$, $R^3$, $R^4$ et $R^5$ sont des restes identiques ou différents et représentent chacun un atome d'hydrogène ou le reste

O
||
$-C-Z-R^6$,

Y et Z sont identiques ou différents et réprésentent chacun un atome d'hydrogène ou de soufre ou un reste N-H ou $CH_2$, et

$R^1$, $R^6$ et $R^7$ sont, identiques ou différents et représentent chacun un reste d'hydrocarbure, éventuellement substitué, comportant jusqu'à 50 atomes de carbone,

à la condition qu'au moins l'un des restes $R^2$, $R^3$, $R^4$, et $R^5$ représente(nt)

O
||
$-C-Z-R^6$,

procédé caractérisé en ce qu'on fait réagir un composé de formule II

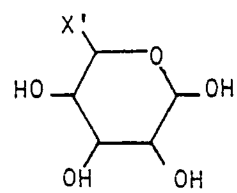

(II)

dans laquelle

X' représente un atome d'hydrogène ou le groupe $CH_2OH$,

sous forme libre, c'est-à-dire non protégée, ou sous forme de dérivés protégés, éventuellement activés, tout d'abord avec une amine $R^7-NH_2$, sous forme libre ou sous forme d'un sel convenable d'addition d'acide, ayant pour $R^7$ le sens indiqué à la revendication 1, puis l'on soumet la glycosylamine ainsi obtenue à acylation sur l'atome d'azote à l'aide d'un dérivé d'acide carboxylique, d'acide carbonique ou d'acide thiocarbonique ou d'un isocyanate, usuels dans les réactions d'acylation, activés, et dont les groupes fonctionnels sont éventuellement protégés, puis l'on enlève, dans le produit réactionnel ainsi obtenu, sélectivement ou entièrement les groupes protecteurs éventuellement présents et, dans une seconde étape opératoire, on fait réagir le produit intermédiaire ainsi obtenu, comportant au moins un groupe hydroxyle libre, c'est-à-dire non protégé, avec un dérivé d'acide carboxylique, d'acide carbonique ou d'acide thiocarbonique ou avec un isocyanate, usuels dans les réactions d'acylation, activés et dont les groupes fonctionnels sont éventuellement protégés, et l'on sépare ou enlève les groupes protecteurs éventuellement présents.

2. Procédé selon la revendication 1, caractérisé en ce que, dans une première étape opératoire, on fait réagir un sucre non bloqué, de formule II, en opérant dans un solvant convenable, éventuellement en présence d'un catalyseur à des températures comprises entre 0°C et 80°C, avec 1 à 10 équivalents de l'amine $R^7-NH_2$ pour obtenir une glycosylamine; dans une seconde étape opératoire, on fait réagir ensuite la glycosylamine avec 1 à 10 équivalents d'un dérivé acylé de formule $R^1-Y-CO-X''$ (dans laquelle $R^1$ et Y ont le sens indiqué à la revendication 1, et X'' représente un atome d'halogène ou un groupe partant usuel dans les réactions d'acylation, avantageusement un reste ester à rôle d'activation, ou un groupe $O-OC-Y-R^1$ ayant le sens ci-dessus pour Y et $R^1$), ou bien avec 1 à 10 équivalents d'un isocyanate de formule $R^1-NCO$, en travaillant dans un solvant organique ou hydro-organique, à des températures comprises entre -30°C et 80°C, éventuellement en

présence d'une base, et, dans une troisième étape opératoire, on fait ensuite réagir le dérivé ainsi obtenu, ayant réagi au niveau de son azote, sous forme protégée ou non protégée, avec 1 à 10 équivalents d'un dérivé acylé de formule

$$\begin{array}{c} O \\ \| \\ R^6\text{-}Z\text{-}C\text{-}X''' \end{array},$$

dans laquelle
$R^6$ et Z ont le sens indiqué à la revendication 1, et
X''' représente un atome d'halogène ou un groupe partant, usuel dans les réactions d'acylation, avantageusement un reste ester à rôle d'activation ou un groupe

$$\begin{array}{c} O \\ \| \\ O\text{-}C\text{-}Z\text{-}R^6 \end{array}$$

dans lequel
les symboles $R^6$ et Z ont le sens ci-dessus,
ou avec 1 à 10 équivalents d'un isocyanate de formule $R^6$-NCO, et l'on travaille alors dans un solvant organique ou hydro-organique, à des températures comprises entre -30°C et +80°C, éventuellement en présence d'une base.

3. Procédé selon la revendication 1, caractérisé en ce qu'on conduit la réaction avec une amine $R^7$-$NH_2$ liquide à la température ambiante en omettant d'utiliser un solvant et en opérant à des températures comprises entre 0 et 100°C.

4. Procédé selon la revendication 3, caractérisé en ce qu'on conduit la réaction avec l'amine $R^7$-$NH_2$ en opérant à des températures de 25 à 70°C.

5. Procédé selon la revendication 3, caractérisé en ce qu'on conduit la réaction avec l'amine $R^7$-$NH_2$ en opérant en présence d'acides minéraux ou d'acides carboxyliques comme catalyseurs.

6. Procédé selon la revendication 5, caractérisé en ce qu'on utilise les catalyseurs en des quantités de 0,001 à 0,05 équivalent, par rapport à l'amine.

7. Procédé selon la revendication 1, caractérisé en ce qu'on conduit la réaction avec une amine $R^7$-$NH_2$ en opérant en présence d'un solvant.

8. Procédé selon la revendication 7, caractérisé en ce qu'on conduit la réaction dans du méthanol, de l'éthanol, du propanol-1, du propanol-2, du tétrahydrofuranne, du dioxanne ou du diméthylformamide.

9. Procédé selon la revendication 7, caractérisé en ce qu'on conduit la réaction à des températures comprises entre -10°C et +120°C.

10. Procédé selon la revendication 2, caractérisé en ce qu'on utilise les composés $R^1$-Y-COX''/$R^6$ZCO-X''' ou $R^1$-NCO/$R^6$-NCO dans 1 à 10 équivalents, par rapport à un système glycosylamine/glycosylamide.